# EUROPEAN PATENT APPLICATION

(11) **EP 4 147 636 A1**
(43) Date of publication of application: **15.03.2023**
(21) Application number: 21810906.4
(22) Date of filing: 30.04.2021
(51) Int. Cl.: A61B 5/055, A61B 5/372, A61B 5/38

(54) **INFORMATION PROCESSING DEVICE AND INFORMATION PROCESSING METHOD**

(30) Priority: 27.05.2020 JP 2020092110
(71) Applicant: Tokyo Institute of Technology, Tokyo 152-8550 (JP)
(72) Inventor: YOSHIMURA, Natsue, Tokyo 152-8550 (JP); KOIKE, Yasuharu, Tokyo 152-8550 (JP)
(74) Representative: Schröer, Gernot H.
(86) International application number: PCT/JP2021/017180
(87) International publication number: WO 2021/241138

(57) **Abstract**

An estimation apparatus 18 is configured to be capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a brain wave of a subject, information on a sound estimated to be recognized by the subject. The estimation apparatus 18 acquires a brain wave of a second subject presented with the predetermined sound. The estimation apparatus 18 estimates, based on a mode of the brain wave acquired, a signal source of the brain wave, from among a plurality of regions in a brain of the second subject. The estimation apparatus 18 inputs the information relating to the signal source estimated to the model and acquires information, output from the model, on a sound estimated to be recognized by the second subject.

## Description

### [TECHNICAL FIELD]

The present disclosure relates to data processing technology and, more particularly, to an information processing apparatus and an information processing method.

### [BACKGROUND ART]

A technology that uses the electroencephalography (hereinafter, also referred to as a "brain wave") or brain activity data for a subject and conducts various analyses relating to the subject is proposed. For example, patent document 1 indicated below proposes a technology of measuring the brain wave of a subject and determining the level of acquisition of a language by the subject based on the measured brain wave. Further, patent document 2 indicated below proposes a technology for identifying a category of an object viewed of imaged by the subject, from the brain activity signal measured while an image of the object is being viewed or imaged, the object being inclusive of an object not used while the decoder is being trained.

[Patent Literature 1] JP2019-128533
[Patent Literature 1] JP2017-076193

### [SUMMARY OF INVENTION]

### [TECHNICAL PROBLEM]

In the related art, the content perceived by the subject is discriminated or selected from a plurality of options prepared in advance, based on the brain wave or brain activity data for the subject. We have therefore thought that it is difficult according to the related art to identify to what degree the subject (inclusive of, for example, subjects in a vegetative state, locked-in patients, etc. who find it difficult to express their intention, as well as healthy individuals) perceived the presented sound (e.g., to identify whether the subject recognized the sound as a language).

The present disclosure addresses the above-described issue, and a purpose thereof is to provide a technology to help discriminate how the presented sound is heard by a person.

### [SOLUTION TO PROBLEM]

An information processing apparatus according to an embodiment of the present disclosure is an apparatus capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a signal indicating a brain activity of a subject, information on a sound estimated to be recognized by the subject, the information processing apparatus including: a brain activity acquisition unit that acquires a signal indicating a brain activity of a second subject presented with the predetermined sound; a signal source estimation unit that estimates, based on a mode of the signal indicating the brain activity acquired by the brain activity acquisition unit, a signal source of the signal indicating the brain activity, from among a plurality of regions in a brain of the second subject; and a recognized sound acquisition unit that inputs the information relating to the signal source estimated by the signal source estimation unit to the model and acquires information, output from the model, on a recognized sound estimated to be recognized by the second subject.

Another embodiment of the present disclosure also relates to an information processing apparatus. The apparatus is an apparatus capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a signal indicating a brain activity of a subject, information on a sound estimated to be recognized by the subject, the information processing apparatus including: a brain activity acquisition unit that acquires a signal indicating a brain activity of a second subject recalling an arbitrary sound; a signal source estimation unit that estimates, based on a mode of the signal indicating the brain activity acquired by the brain activity acquisition unit, a signal source of the signal indicating the brain activity, from among a plurality of regions in a brain of the second subject; and a recognized sound acquisition unit that inputs the information relating to the signal source estimated by the signal source estimation unit to the model and acquires information, output from the model, on a sound estimated to be recalled by the second subject.

Still another embodiment of the present disclosure relates to an information processing method. The information processing method is implemented on a computer capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a signal indicating a brain activity of a subject, information on a sound estimated to be recognized by the subject, the method including: acquiring a signal indicating a brain activity of a second subject presented with the predetermined sound; estimating, based on a mode of the signal indicating the brain activity acquired, a signal source of the signal indicating the brain activity, from among a plurality of regions in a brain of the second subject; and inputting the information relating to the signal source estimated to the model and acquiring information, output from the model, on a recognized sound estimated to be recognized by the second subject.

Still another embodiment of the present disclosure also relates to an information processing method. The method is implemented on a computer capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a signal indicating a brain activity of a subject, information on a sound estimated to be recognized by the subject, the method including: acquiring a signal indicating a brain activity of a second subject recalling an arbitrary sound; estimating, based on a mode of the signal indicating the brain activity acquired, a signal source of the signal indicating the brain activity, from among a plurality of regions in a brain of the second subject; and inputting the information relating to the signal source estimated to the model and acquiring information, output from the model, on a sound estimated to be recalled by the second subject.

Optional combinations of the aforementioned constituting elements, and implementations of the present disclosure in the form of systems, programs, recording mediums storing programs, etc. may also be practiced as additional modes of the present disclosure.

### [ADVANTAGEOUS EFFECTS OF INVENTION]

According to the present disclosure, it is possible to help discriminate how the presented sound is heard by a person or discriminate a sound that comes to a person's mind.

### [BRIEF DESCRIPTION OF DRAWINGS]

Fig. 1 shows an outline of an estimation system according to the embodiment;
Fig. 2 shows an outline of an estimation system according to the embodiment;
Fig. 3 is a block diagram showing functional blocks of the model generation apparatus of Fig. 2;
Fig. 4 shows a network configuration of the sound estimation model;
Fig. 5 is a block diagram showing functional blocks of the estimation apparatus of Fig. 2;
Fig. 6 shows an example of comparison image;
Fig. 7 schematically shows a method of generating intra-brain information;
Fig. 8 shows an example of intra-brain information;
Fig. 9 shows an example of intra-brain information; and
Figs. 10A and 10B are graphs showing results of experiments.

### [DESCRIPTION OF EMBODIMENTS]

A summary will be given before describing configuration of the estimation system of the embodiment.

In the embodiment, a technology is proposed to replicate how the presented sound is heard by a person and to help discrimination, by using a mathematical model (in the embodiment, a neural network; hereinafter, also referred to as a "sound estimation model") built by machine learning. In the embodiment, the brain wave (scalp brain wave) is used as a signal indicating the brain activity of a subject. Magnetoencephalography may be used as a signal indicating the brain activity of a subject. Alternatively, results of measurement by a near-infrared spectroscopy (NIRS) encephalometer may be used. The details will be described later.

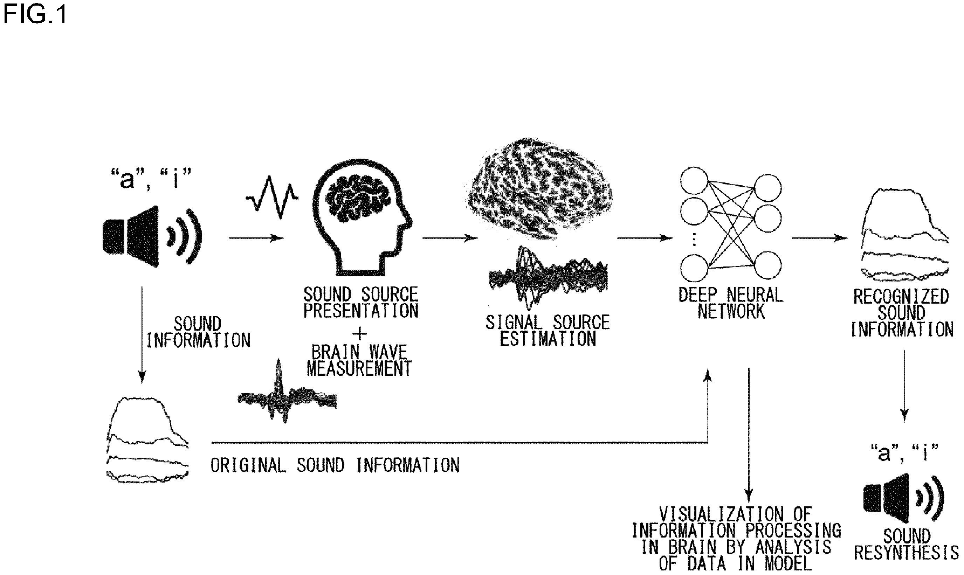

Fig. 1 shows an outline of an estimation system according to the embodiment. In the learning phase, the estimation system of the embodiment lets the first subject hear a predetermined sound such as "Ah" and "Ee" (hereinafter, also referred to as an "original sound") to measure the brain wave of the first subject and estimate a signal source of the brain wave. The estimation system builds, based on signal source information relating to the first subject and original sound information, a sound estimation model configured to receive signal source information and to output information on a sound (hereinafter, also referred to as a "recognized sound") estimated to be recognized by the person presented with the original sound. The original sound may not be a language sound. The original sound may be, for example, an animal call or a mechanical sound that does not have any meaning.

In the estimation phase, the estimation system of the embodiment also lets the second subject hear the above original sound to measure the brain wave of the second subject and estimate a signal source of the brain wave. The estimation system inputs signal source information relating to the second subject to the sound estimation model and acquires, from the sound estimation model, information on a sound (recognized sound) estimated to be recognized by the second subject presented with the original sound. The estimation system can make it clear how the original sound is heard by the second subject by playing back the recognized sound.

The first subject and the second subject in the embodiment are the same person. For example, the first subject and the second subject may be one healthy individual (a person who can understand the sound and express his or her intention). Alternatively, the first subject and the second subject may be a person who finds it difficult to express their intention (i.e., communicate) such as a person having hearing difficulties, a person in a vegetative state, a locked-in patient, etc. In a variation, the first subject and the second subject may be different persons (described later). The "subject" in the embodiment can be said to be a "participant" in the experiment.

In the estimation phase, the estimation system also analyzes the data in the sound estimation model receiving the signal source information relating to the second subject to visualize information processing in the brain. More specifically, the estimation system generates intra-brain information indicating the impact of each of a plurality of regions in the brain the second subject on the recognized sound. This makes it possible to visualize for each individual which region in the brain is used and at what time point it is used.

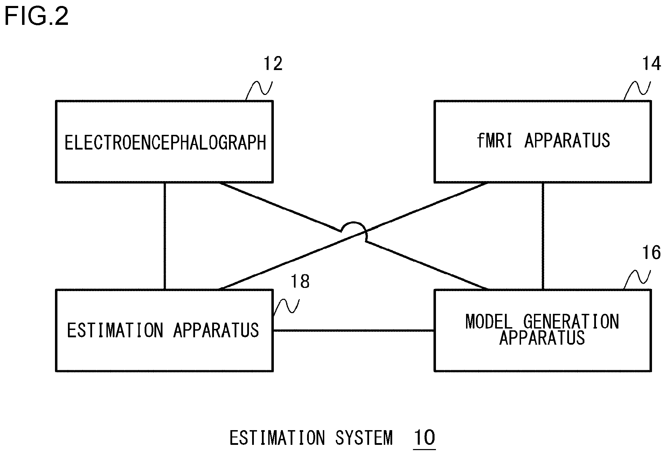

Fig. 2 shows an outline of an estimation system 10 according to the embodiment. The estimation system 10 is an information processing system provided with an electroencephalograph 12, a functional magnetic resonance imaging (fMRI) apparatus 14, a model generation apparatus 16, and an estimation apparatus 18. In the embodiment, the apparatuses of Fig. 2 are connected via a communication network such as LAN, and data are transmitted and received online. In a variation, data may be exchanged offline via a recording medium such as a USB storage.

The electroencephalograph 12 detects a signal (hereinafter, "brain wave signal") indicating the brain wave of the subject via a plurality of electrodes (i.e., sensors) placed on the scalp of the subject. The number of electrodes can be modified as appropriate. In the embodiment, 30 electrodes are used. In other words, the electroencephalograph 12 detects the brain wave signals on 30 channels. The electroencephalograph 12 outputs data indicating the brain wave signals on 30 channels thus detected to the model generation apparatus 16 in the learning phase. In the estimation phase, the electroencephalograph 12 outputs the data to the estimation apparatus 18.

The data indicating the brain wave signal may be, for example, data that maps the time and the amplitude. Alternatively, the data may map the frequency to the power spectrum density, i.e., may indicate the frequency characteristic. The electroencephalograph 12 may amplify the brain wave signal or eliminate the noise from the brain wave signal by a publicly known method.

The fMRI apparatus 14 is an apparatus that visualizes the hemodynamic reaction relating to the brain activity by using magnetic resonance imaging (MRI). The fMRI apparatus 14 outputs brain activity data, which is data indicating a brain site activated in the brain of the subject, to the model generation apparatus 16 in the learning phase. In the estimation phase, the fMRI apparatus 14 outputs the data to the estimation apparatus 18. The brain activity data can be said to be data indicating the signal source of the brain wave based on actual measurement.

The model generation apparatus 16 is an information processing apparatus (i.e., a computer device) that generates a sound estimation model. The estimation apparatus 18 is an information processing apparatus that estimates the sound recognized by the subject by using the sound estimation model generated by the model generation apparatus 16. The detailed configuration of these apparatuses will be described later.

The number of housings for the apparatuses of Fig. 1 is not limited. For example, at least one apparatus shown in Fig. 1 may be implemented by coordinating a plurality of information processing apparatuses. Alternatively, the functions of a plurality of apparatuses shown in Fig. 1 may be implemented by a single information processing apparatus. For example, the function of the model generation apparatus 16 and the function of the estimation apparatus 18 may be implemented in a single information processing apparatus.

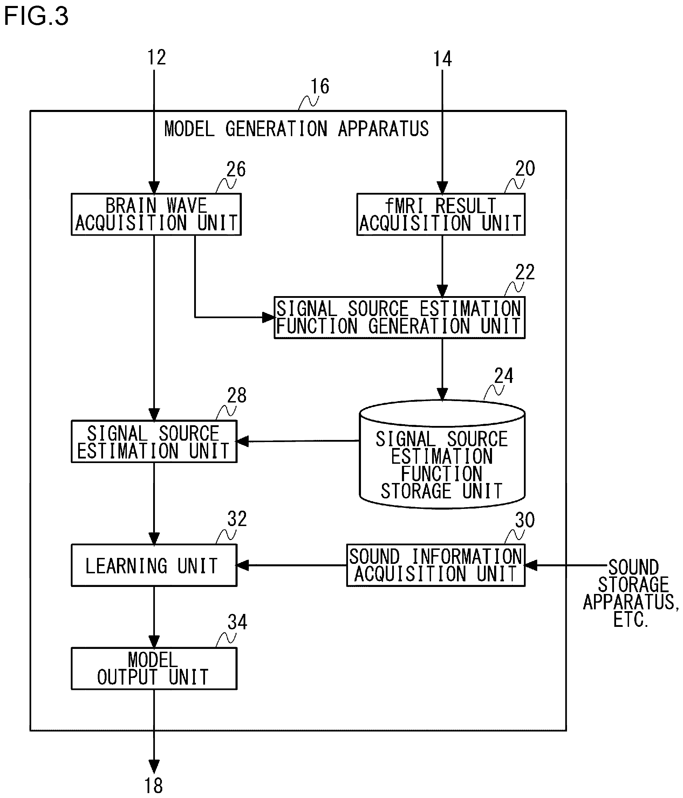

Fig. 3 is a block diagram showing functional blocks of the model generation apparatus 16 of Fig. 2. The model generation apparatus 16 is provided with a fMRI result acquisition unit 20, a signal source estimation function generation unit 22, a signal source estimation function storage unit 24, a brain wave acquisition unit 26, a signal source estimation unit 28, a sound information acquisition unit 30, a learning unit 32, and a model output unit 34.

The blocks depicted in the block diagram of this specification are implemented in hardware such as elements or mechanical devices such as a CPU and a memory of a computer, and in software such as a computer program. Fig. 2 depicts functional blocks implemented by the cooperation of these elements. Therefore, it will be understood by those skilled in the art that the functional blocks may be implemented in a variety of manners by a combination of hardware and software.

A computer program implementing the functions of at least some of the plurality of functional blocks shown in Fig. 3 may be stored in a predetermined recording medium, and the computer program may be installed in the storage of the model generation apparatus 16 via the recording medium. Alternatively, the above computer program may be downloaded from a server via a communication network and installed in the storage of the model generation apparatus 16. The CPU of the model generation apparatus 16 may cause the plurality of functional blocks shown in Fig. 3 to exhibit their functions by reading the computer program into the main memory and running the computer program.

The fMRI result acquisition unit 20 acquires the brain activity data for the subject (the first subject above) input from the fMRI apparatus 14. In the embodiment, "data acquisition" is inclusive of receiving data transmitted from outside and of storing the received data in a memory or a storage.

In the embodiment, a plurality of sites (it can be said to be "regions") resulting from dividing the brain surface (e.g., the brain cortex) into predetermined sizes are defined. In the embodiment, 100 sites are defined. These plurality of sites may include, for example, publicly known sites such as amygdala, insular cortex, and anterior cingulate cortex or may include sites resulting from further dividing the publicly known site(s). The signal source estimation function generation unit 22 refers to the brain wave data and generates a signal source estimation function for estimating a signal source of the brain wave.

The signal source estimation function of the embodiment is a function that receives the brain wave data on 30 channels and outputs data indicating whether each of the 100 brain sites is active or not active. Stated otherwise, the signal source estimation function is a function that outputs data indicating whether each brain site is a signal source. The signal source estimation function may be a matrix of 30x100 that weights each of the 100 brain sites as a signal source, based on the brain wave data on 30 channels.

The above process of the signal source estimation function generation unit 22 is realized by variational Bayesian multimodal encephalography (VBMEG), publicly known software provided by Advance Telecommunications Research Institute International. VBMEG is software for estimating a signal source by estimating the cortical current of the brain based on the brain wave data. The brain wave data may be data indicating the waveform of the brain wave, data indicating the time-dependent transition of the amplitude, or data indicating the frequency characteristic of the brain wave.

More specifically, the signal source estimation function generation unit 22 causes the VBMEG to generate a signal source estimation function by inputting, to the predetermined application programming interface (API) provided by the VBMEG, (1) image data indicating the structure of the brain imaged by fMRI, (2) brain activity data measured by fMRI, (3) data indicating the position of placement of the electrodes on the scalp, and (4) data for the brain wave signal acquired by the brain wave acquisition unit 26.

The signal source estimation function generation unit 22 stores the signal source estimation function thus generated in the signal source estimation function storage unit 24. The signal source estimation function storage unit 24 is a storage area for storing the signal source estimation function generated by the signal source estimation function generation unit 22.

The brain wave acquisition unit 26 can be said to be a brain activity acquisition unit. The brain wave acquisition unit 26 acquires, as a signal indicating the brain activity of the subject, data for the brain wave signal input from the electroencephalograph 12. The brain wave acquisition unit 26 outputs the acquired data for the brain wave signal to the signal source estimation function generation unit 22 and the signal source estimation unit 28.

The signal source estimation unit 28 estimates one or more signal sources of the brain wave from among a plurality of brain sites of the subject (in this case, the first subject) based on the mode of the brain wave, acquired by the brain wave acquisition unit 26, as a signal indicating the brain activity of the subject. The signal source estimation unit 28 may estimate one or more signal sources of the brain wave, based on the temporal-spatial information relating to the brain wave of the first subject (e.g., the shape of the waveform of the brain wave, the position on the scalp where the brain wave is measured, the position of a plurality of signal sources, the irregularity on the brain cortex (so-called wrinkles of the brain), the conductivity of the tissue between the scalp and the brain).

In the embodiment, the signal source estimation unit 28 acquires data relating to one or more signal sources (hereinafter, "signal source data") as an output of the signal source estimation function, by inputting the brain wave data on 30 channels to the signal source estimation function stored in the signal source estimation function storage unit 24. The signal source estimation unit 28 delivers the signal source data thus acquired as a estimation result to the learning unit 32.

The signal source data output by the signal source estimation unit 28 is data indicating, for each (candidate) of the plurality of signal sources, the time-dependent transition of the signal intensity (the magnitude of the electric current) of the brain wave output from each signal source. More specifically, the signal source data is data indicating the signal intensity of the brain wave output from each of 100 predefined signal sources at 77 time points within 0.3 seconds. The same is true of the signal source data output from the signal source estimation unit 50 described later.

The sound information acquisition unit 30 acquires the data for the original sound from an external storage apparatus, etc. presented to the subject, for which the brain wave is measured by the electroencephalograph 12 and the brain activity is measured by the fMRI apparatus 14. The sound information acquisition unit 30 generates original sound information, which is information indicating the time-dependent transition of a plurality of feature amounts (acoustic feature amounts) relating to the original sound, by applying a publicly known sound analysis (e.g., mel-cepstrum analysis) to the data for the original sound acquired from outside. As shown in Fig. 1, the original sound information of the embodiment is time series data for five feature amounts.

The learning unit 32 can be said to be a model generation unit and generates a sound estimation model by a publicly known machine learning scheme (in the embodiment, deep learning), using, as training data, the original sound information acquired by the sound information acquisition unit 30 and the signal source data estimated by the signal source estimation unit 28. The sound estimation model is a convolutional neural network that receives the signal source data for the brain wave of the subject presented with the sound and outputs information on the sound estimated to be recognized by the subject (recognized sound). The learning unit 32 may generate the sound estimation model by using a publicly known library or a framework such as Keras.

In a variation, the process of machine learning such as deep learning performed by the learning unit 32 (e.g., generation of the sound estimation model) may be performed in a computer on a cloud (cloud computer). In this case, the model generation apparatus 16 may deliver the training data to the cloud computer, acquire the result of learning by the cloud computer (e.g., the sound estimation model), and provide the result to the estimation apparatus 18 via a communication network The estimation apparatus 18 may use the result of learning by the cloud computer to estimate the recognized sound of the subject.

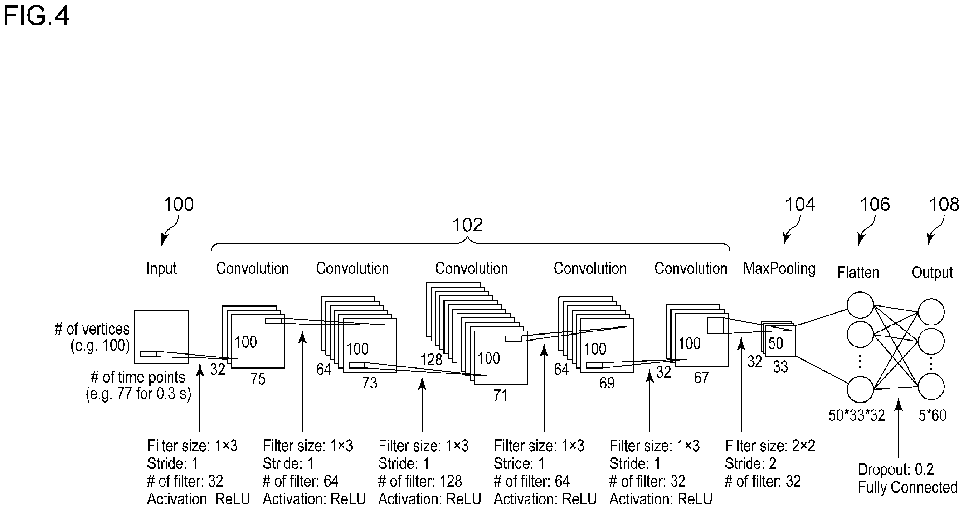

Fig. 4 shows a network configuration of the sound estimation model. The sound estimation model includes an input layer 100, a plurality of convolutional layers 102, a maximum pooling layer 104, a fully connected layer 106, and an output layer 108. Time series data for the signal intensity (the signal intensity at 77 time points) of each of the 100 signal sources indicated by the signal source data is input to the input layer 100. The output layer 108 outputs time series data for the plurality of feature amounts relating to the recognized sound. In the example of Fig. 4, the output layer 108 outputs recognized sound information indicating the values of the five feature amounts at 60 time points.

Referring back to Fig. 3, the model output unit 34 transmits the data for the sound estimation model generated by the learning unit 32 to the estimation apparatus 18 to cause the model storage unit 40 of the estimation apparatus 18 to store the data for the sound estimation model.

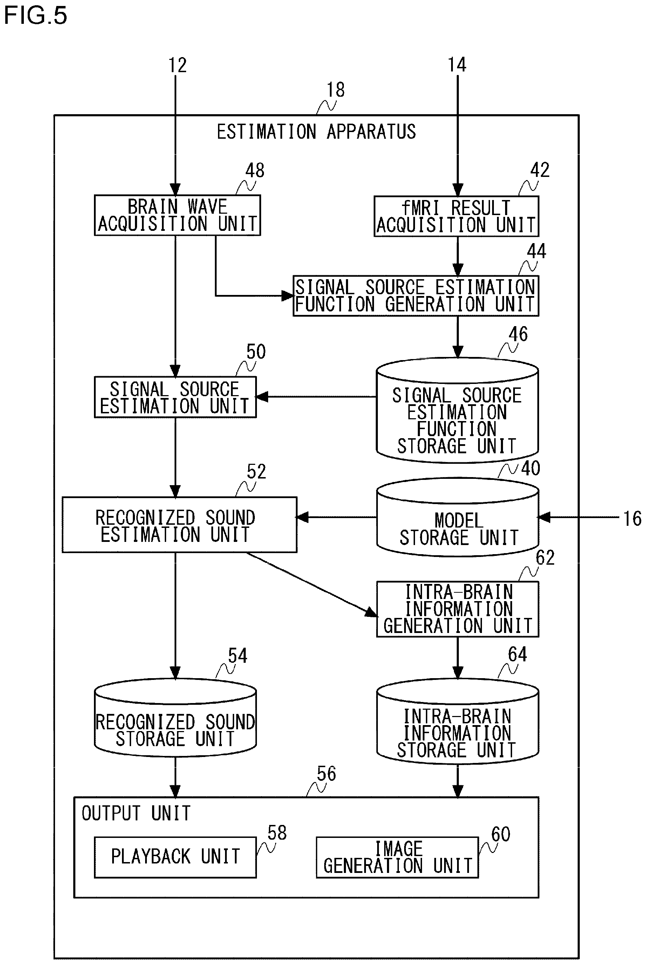

Fig. 5 is a block diagram showing functional blocks of the estimation apparatus 18 of Fig. 2. The estimation apparatus 18 is provided with a model storage unit 40, a fMRI result acquisition unit 42, a signal source estimation function generation unit 44, a signal source estimation function storage unit 46, a brain wave acquisition unit 48, a signal source estimation unit 50, a recognized sound estimation unit 52, a recognized sound storage unit 54, an output unit 56, an intra-brain information generation unit 62, an intra-brain information storage unit 64.

A computer program implementing at least some of the plurality of functional blocks shown in Fig. 5 may be stored in a recording medium and installed in the storage of the estimation apparatus 18 via the recording medium. Alternatively, the computer program may be downloaded from a server via a communication network and installed in the storage of the estimation apparatus 18 via a communication network. The CPU of the estimation apparatus 18 may cause the plurality of functional blocks shown in Fig. 5 to exhibit their functions by reading the computer program into the memory and running the computer program.

The model storage unit 40 stores the data for the sound estimation model transmitted from the model generation apparatus 16. In a variation, the model generation apparatus 16 may be provided with a storage unit for storing the sound estimation model. In this case, the estimation apparatus 18 may refer to the sound estimation model stored in the model generation apparatus 16 via a communication network. In other words, the estimation apparatus 18 may access a local or remote storage unit that stores the sound estimation model. Stated otherwise, the estimation apparatus 18 may be configured to refer to the sound estimation model stored in a local or remote storage unit.

The fMRI result acquisition unit 42, the signal source estimation function generation unit 44, the signal source estimation function storage unit 46, the brain wave acquisition unit 48 correspond to the fMRI result acquisition unit 20, the signal source estimation function generation unit 22, the signal source estimation function storage unit 24, and the brain wave acquisition unit 26 of the model generation apparatus 16 already described. Therefore, a description of the features of the fMRI result acquisition unit 42, the signal source estimation function generation unit 44, the signal source estimation function storage unit 46, and the brain wave acquisition unit 48 common to those of the corresponding functional blocks will be omitted. Those features that are different from those of the corresponding functional blocks will be highlighted.

The fMRI result acquisition unit 42 acquires the brain activity data, input from the fMRI apparatus 14, for the subject for which the recognized sound is estimated (i.e., the second subject presented with the original sound). The brain wave acquisition unit 48 can be said to be a brain activity acquisition unit. The brain wave acquisition unit 48 acquires, as a signal indicating the brain activity of the subject, data for the brain wave signal of the second subject presented with the original sound.

The signal source estimation function generation unit 44 generates a signal source estimation function for estimating a signal source of the brain wave from the data for the brain wave signal of the second subject. The signal source estimation function storage unit 46 stores the signal source estimation function relating to the second subject. In the embodiment, the first subject and the second subject are the same person. Therefore, the estimation apparatus 18 may use the signal source estimation function generated by the model generation apparatus 16 (i.e., generated in the learning phase), and the signal source estimation function storage unit 46 may store the signal source estimation function generated by the model generation apparatus 16.

The signal source estimation unit 50 estimates one or more signal sources of the brain wave from among a plurality of brain sites of the subject (in this case, the second subject) based on the mode of the brain wave, acquired by the brain wave acquisition unit 48, as a signal indicating the brain activity of the subject. The signal source estimation unit 50 estimates one or more signal sources of the brain wave, based on the temporal-spatial information relating to the brain wave of the second subject (e.g., the shape of the waveform of the brain wave, the position on the scalp where the brain wave is measured, the position of a plurality of signal sources, the irregularity on the brain cortex (so-called wrinkles of the brain), the conductivity of the tissue between the scalp and the brain).

In the embodiment, the signal source estimation unit 50 acquires signal source data relating to one or more signal sources as an output of the signal source estimation function, by inputting the brain wave data on 30 channels to the signal source estimation function stored in the signal source estimation function storage unit 46. The signal source estimation unit 50 delivers the signal source data thus acquired as an estimation result to the recognized sound estimation unit 52.

The recognized sound estimation unit 52 can be said to be a recognized sound acquisition unit. The recognized sound estimation unit 52 reads the data for the sound estimation model stored in the model storage unit 40 into the main memory and inputs the signal source data estimated by the signal source estimation unit 50 to the input layer of the sound estimation model. The recognized sound estimation unit 52 acquires time series data (the recognized sound information described above) output from the output layer of the sound estimation model and relating to the feature amount of the recognized sound estimated to be recognized by the second subject.

The recognized sound storage unit 54 stores the recognized sound information acquired by the recognized sound estimation unit 52. The recognized sound estimation unit 52 may store the recognized sound information in the recognized sound storage unit 54. The recognized sound storage unit 54 may acquire the recognized sound information from the recognized sound estimation unit 52 and store the recognized sound information. Further, the recognized sound storage unit 54 may be a volatile storage area or a non-volatile storage area.

The output unit 56 outputs the recognized sound information acquired by the recognized sound estimation unit 52 outside. In the embodiment, the output unit 56 outputs the recognized sound information stored in the recognized sound storage unit 54 outside. The output unit 56 includes a playback unit 58 and an image generation unit 60. The playback unit 58 plays back the sound indicated by the recognized sound information by applying a publicly known sound synthesis process to the recognized sound information acquired by the recognized sound estimation unit 52 and, in the embodiment, stored in the recognized sound storage unit 54 and causes a speaker (not shown) to output the playback sound.

The image generation unit 60 acquires the data for the sound (i.e., the original sound) presented to the second subject from an external storage apparatus (not shown). The image generation unit 60 subjects the original sound to publicly known mel-cepstrum analysis to generate time series data ("original sound information") indicating the transition of the plurality of feature amounts of the original sound. Further, the image generation unit 60reads the recognized sound information stored in the recognized sound storage unit 54, i.e., the time series data indicating the transition of the plurality of feature amounts of the recognized sound. The image generation unit 60 generates an image (hereinafter, also referred to as "comparison image") showing both the waveform of the original sound and the waveform of the recognized sound, based on the original sound information and the recognized sound information.

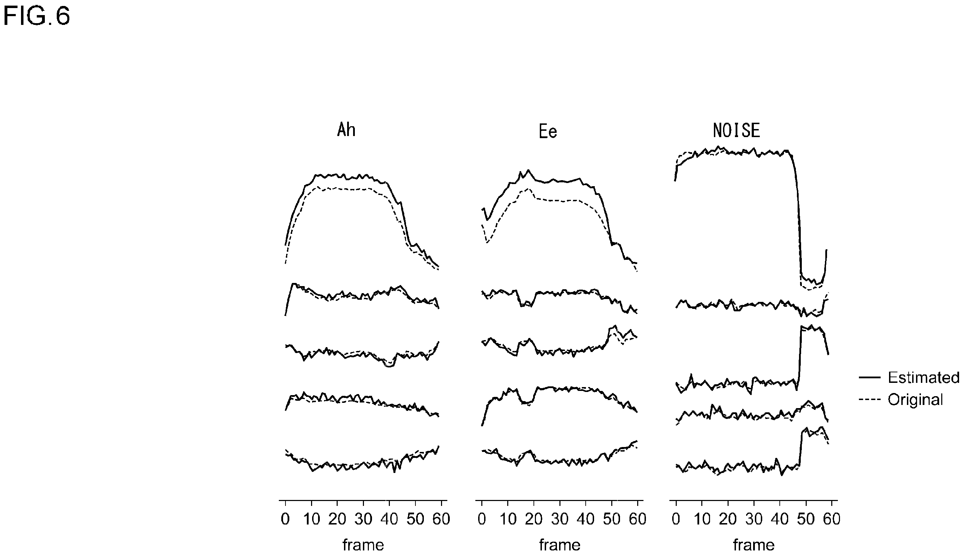

Fig. 6 shows an example of comparison image. The figure shows the waveform of the original sound in a broken line and shows the waveform of the recognized sound in a solid line, for each of "Ah", "Ee", and noise. In the example of Fig. 6, the image generation unit 60 generates a comparison image for each of "Ah", "Ee", and noise, showing graphs of respective feature amounts superimposed on one other.

The image generation unit 60 may store the data for the comparison image thus generated in a local or remote storage unit. Alternatively, the image generation unit 60 may output the data for the comparison image thus generated to a display device (not shown) to cause the display apparatus to display the comparison image.

The intra-brain information generation unit 62 refers to the information recorded in the sound estimation model to which the signal source data is input by the recognized sound estimation unit 52. The intra-brain information generation unit 62 generates intra-brain information, which is information indicating the impact of each of the plurality of regions in the brain of the second subject on the recognized sound. The intra-brain information generation unit 62 stores the intra-brain information thus generated in the intra-brain information storage unit 64. The intra-brain information storage unit 64 is a storage area for storing the intra-brain information generated by the intra-brain information generation unit 62. The output unit 56 outputs the intra-brain information stored in the intra-brain information storage unit 64 to a local or remote storage apparatus to cause it to store the intra-brain information. Alternatively, the output unit 56 outputs the intra-brain information to a local or remote display apparatus to cause it to display the intra-brain information.

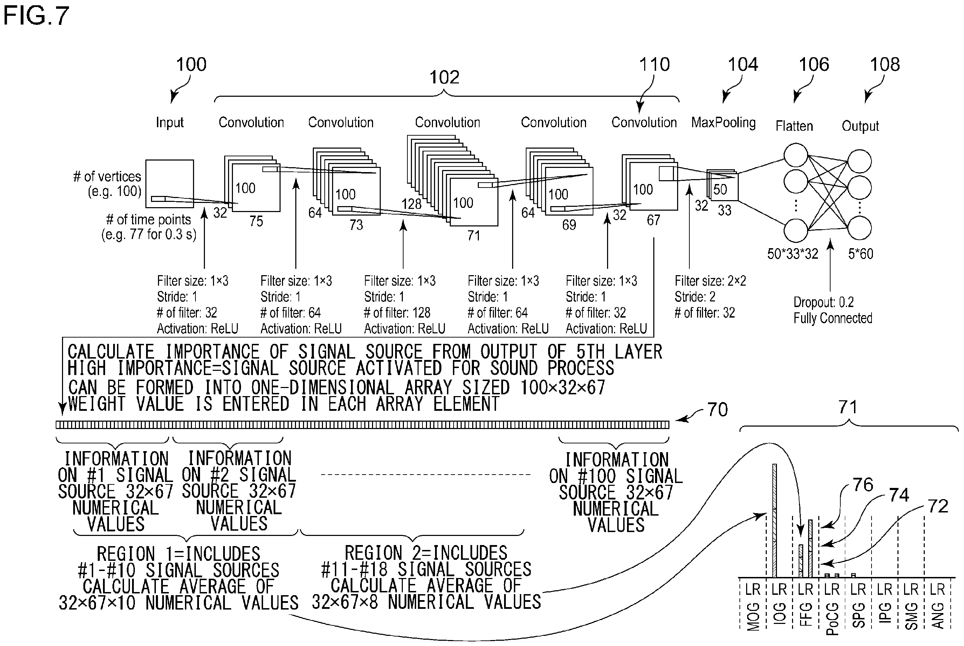

Fig. 7 schematically shows a method of generating intra-brain information. As already described, the sound estimation model includes the input layer 100, the plurality of convolutional layers 102, the maximum pooling layer 104, the fully connected layer 106, and the output layer 108. The plurality of convolutional layers 102 are configured to extract a signal source having a large impact on the recognized sound through a plurality of filtering steps without an intervening pooling layer. A convolutional layer 110 is the layer positioned at the end of a series of plurality of convolutional layers 102. Information relating to the impact (which can be said to be a weight) of each of the plurality of regions in the brain (i.e., the plurality of signal sources) on the recognized sound is most clearly recorded in the convolutional layer 110.

The intra-brain information generation unit 62 refers to the sound estimation model to which the signal source data is input by the recognized sound estimation unit 52 and which outputs the recognized sound information. The intra-brain information generation unit 62 reads the information recorded in the convolutional layer 110, i.e., the information (which can be said to be weight information) output from the convolutional layer 110, to generate an array 70. Fig. 7 illustrates the array 70 as a one-dimensional array of 100 signal sources × 32 channels × 67 time points.

The intra-brain information generation unit 62 stores the correspondence between each of the plurality of regions in the brain (e.g., MOG, IOG, FFG, etc. shown in Fig. 7) and one or more signal sources in advance. Regions of the same name in the left brain and the right brain are dealt with as different regions. For example, L FFG in Fig. 7 represents FFG in the left brain, and R FFG in Fig. 7 represents FFG in the right brain. The intra-brain information generation unit 62 generates, for each of the plurality of regions in the bran, intra-brain information indicating the magnitude of impact that each region in the brain exercises on the recognized sound, based on the information relating to one or more signal sources corresponding to the region.

More specifically, the intra-brain information generation unit 62 calculates, for each region in the brain, an average value of 32×67 numerical values (values indicating the magnitude of impact on the recognized sound) per one signal source, the average value representing information relating to one or more corresponding signal sources. The intra-brain information generation unit 62 stores the above average value for each region in the brain in the intra-brain information as a value indicating the impact of each region in the brain on the recognized sound.

The intra-brain information 71 of Fig. 7 indicates the impact that each region in the brain exercises on the recognized sound in the case the original sound is "Ah" by the length of an indicator 72. Further, the intra-brain information 71 indicates the impact that each region in the brain exercises on the recognized sound in the case the original sound is "Ee" by the length of an indicator 74. Further, the intra-brain information 71 indicates the impact that each region in the brain exercises on the recognized sound in the case the original sound is noise (white noise) by the length of an indicator 76. The longer the indicator 72, the indicator 74, and the indicator 76, the more actively the corresponding sound is being processed.

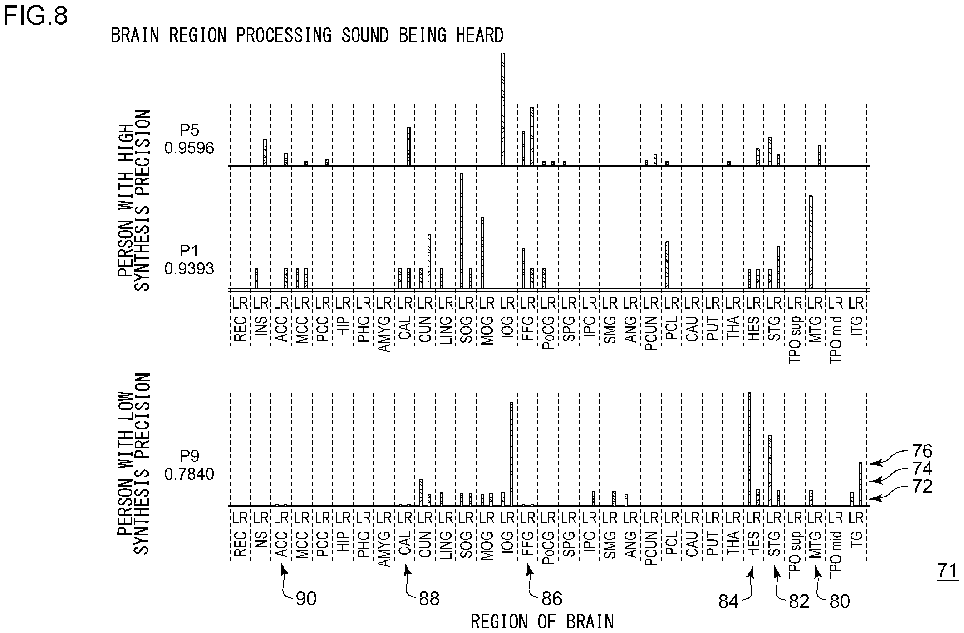

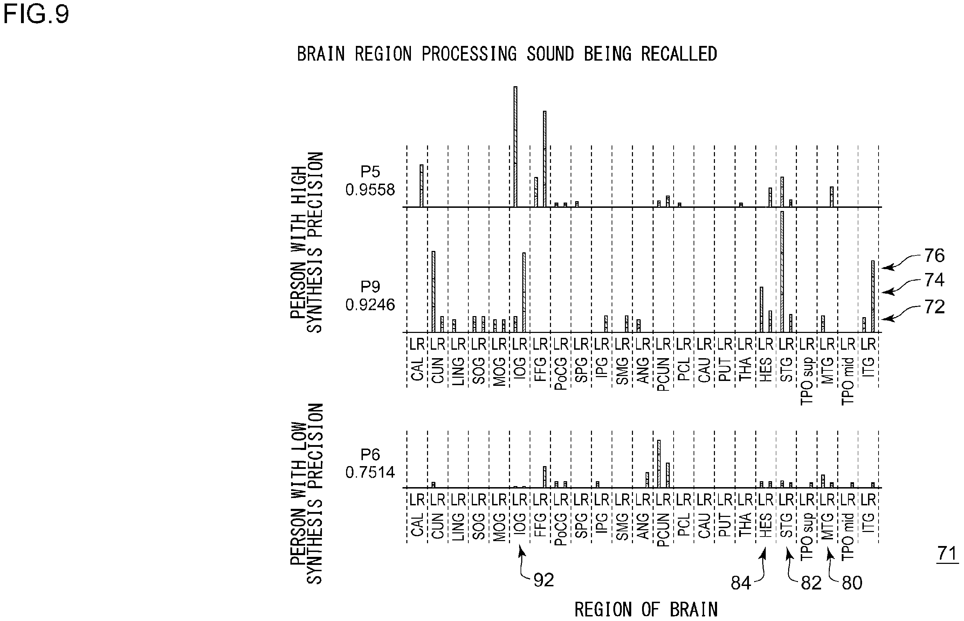

Fig. 8 and Fig. 9 show examples of intra-brain information. Fig. 8 shows the intra-brain information 71 generated when the second subject hears the original sound such as "Ah" and "Ee", i.e., the intra-brain information 71 showing regions in the brain processing the sound when the original sound is heard. Meanwhile, Fig. 9 shows the intra-brain information 71 generated when the second subject remembers the original sound heard in the past, i.e., the intra-brain information 71 showing regions in the brain processing the sound when the original sound heard in the past is being remembered. The indicator 72, the indicator 74, and the indicator 76 of Fig. 8 and Fig. 9 correspond to the indicator 72, the indicator 74, and the indicator 76 of Fig. 7.

A comparison between the intra-brain information 71 of Fig. 8 and the intra-brain information 71 of Fig. 9 reveals the difference in the intra-brain process between when the sound is being heard and when the sound is being remembered. For example, a region 80, a region 82, and a region 84 tend to be used both when the sound is being heard and when the sound is being remembered. Meanwhile, a region 86, a region 88, a region 90, and a region 92 are considered to be regions that are more needed or less needed depending on whether the sound is being heard or being remembered.

The intra-brain information 71 visualizes in real time which region in the brain is active when the sound is being heard and when the sound is being remembered. This can visualize the variation in the brain activity due to a difference in awareness of the subject hearing the sound. For example, it is possible to know which region in the brain has a week activity by visualizing the brain activity of a person hard of hearing by the intra-brain information 71. Further, information helpful for improvement of the auditory function can be obtained by visualizing the brain activity of the subject in real time by the intra-brain information 71.

A description will be given of the operation of the estimation system 10 having the above-described configuration.

First, a description will be given of the operation of the learning phase in which the model generation apparatus 16 plays the central role. The fMRI apparatus 14 measures the brain activity of the first subject and outputs the measured brain activity data to the model generation apparatus 16. The fMRI result acquisition unit 20 of the model generation apparatus 16 acquires the brain activity data, and the signal source estimation function generation unit 22 starts the VBMEG to generate the signal source estimation function. More specifically, the signal source estimation function generation unit 22 generates the signal source estimation function for the first subject by calling a publicly known function provided by the VBMEG, using the brain structure data, electrode positions, and brain activity data for the first subject as parameters. The signal source estimation function generation unit 22 stores the signal source estimation function for the first subject in the signal source estimation function storage unit 24.

The electroencephalograph 12 measures the brain wave of the first subject via the electrodes placed on the scalp of the first subject presented with the original source (e.g., "Ah", "Ee", or noise). The electroencephalograph 12 outputs the brain wave data for the first subject to the model generation apparatus 16. The brain wave acquisition unit 26 of the model generation apparatus 16 acquires the brain wave data for the first subject, and the signal source estimation unit 28 estimates the signal source of the brain wave of the first subject in accordance with the brain wave data for the first subject and the signal source estimation function stored in the signal source estimation function storage unit 24.

The learning unit 32 generates training data that maps the original sound presented to the first subject to the signal source data for the brain wave of the first subject and performs machine learning based on the training data. The learning unit 32 generates a sound estimation model that receives the signal source data as an input and outputs recognized sound information on the recognized sound estimated to be recognized by the subject presented with the original sound. The model output unit 34 transmits the data for the sound estimation model to the estimation apparatus 18 to cause the model storage unit 40 of the estimation apparatus 18 to store the data.

A description will now be given of the operation in the estimation phase in which the estimation apparatus 18 plays the central role. The fMRI apparatus 14 measures the brain activity of the second subject and outputs the measured brain activity data to the estimation apparatus 18. The fMRI result acquisition unit 42 of the estimation apparatus 18 acquires the brain activity data, and the signal source estimation function generation unit 44 starts the VBMEG to generate the signal source estimation function. More specifically, the signal source estimation function generation unit 44 generates the signal source estimation function for the second subject by calling a publicly known function provided by the VBMEG, using the brain structure data, electrode positions, and brain activity data for the second subject as parameters. The signal source estimation function generation unit 44 stores the signal source estimation function for the second subject in the signal source estimation function storage unit 46.

The electroencephalograph 12 measures the brain wave of the second subject via the electrodes placed on the scalp of the second subject presented with the original source. The electroencephalograph 12 outputs the brain wave data for the second subject to the estimation apparatus 18. The brain wave acquisition unit 48 of the estimation apparatus 18 acquires the brain wave data for the second subject, and the signal source estimation unit 50 estimates the signal source of the brain wave of the second subject in accordance with the brain wave data for the second subject and the signal source estimation function stored in the signal source estimation function storage unit 46.

The recognized sound estimation unit 52 reads the sound estimation model stored in the model storage unit 40. The recognized sound estimation unit 52 inputs the signal source data for the second subject to the sound estimation model and acquires the recognized sound information relating to the second subject output from the sound estimation model. The recognized sound estimation unit 52 stores the recognized sound information relating to the second subject in the recognized sound storage unit 54. The playback unit 58 plays back the sound indicated by the recognized sound information stored in the recognized sound storage unit 54. The image generation unit 60 generates a comparison image showing the waveform of the original sound and the waveform of the recognized sound indicated by the recognized sound information stored in the recognized sound storage unit 54 arranged side by side. The image generation unit 60 causes a local or remote display apparatus to display the generated comparison image.

The intra-brain information generation unit 62 refers to the information recorded in the sound estimation model to which the signal source data for the brain wave of the second subject is input and generates intra-brain information indicating the impact of each of the plurality of regions in the brain of the second subject on the recognized sound. The intra-brain information generation unit 62 stores the generated intra-brain information in the intra-brain information storage unit 64. The output unit 56 outputs the intra-brain information recorded in the intra-brain information storage unit 64 to an external appliance (storage apparatus, display apparatus, etc.).

The estimation system 10 of the embodiment generates information on the sound itself estimated to be recognized by the subject (the second subject). This makes it possible to examine whether the subject is recognizing the sound accurately in the case the subject is a healthy individual. It is also possible to examine whether the sound is being heard or recognized in the brain as a language as well as being heard, in the case the subject is an individual in a vegetative state, a locked-in patient, an infant, or the like who finds it impossible or difficult to express his or her intention. In further accordance with the estimation system 10, it is possible to help discriminate how the presented sound is being heard or to what degree the sound is recognized by the subject, by playing back the recognized sound of the subject (the second subject) or showing the waveform of the original sound and the waveform of the recognized sound in an easily comparable manner.

In further accordance with the estimation system 10, it is possible to present the original sound to the subject (the second subject) wearing a hearing aid and examine the recognized sound of the subject. This can facilitate development of highquality hearing aids based on the aural perception of users. Further, the estimation apparatus 18 makes it possible to check the recognized sound recognized by the subject who is remembering the original sound and so facilitates determination of the recognition function of the subject.

In further accordance with the estimation system 10, the activity situation of each region in the brain of the subject can be visualized (e.g., the intra-brain information 71 of Fig. 8, Fig. 9). By building a stock of the intra-brain information 71 for a large number of individuals, the difference between persons losing hearing (e.g., aged persons and persons who use earphones excessively) and persons having a normal auditory function can be visualized at the level of brain region. Further, establishment and evaluation of a training method for maintaining the healthy auditory function can be facilitated. Further, it is possible, once the training method is established, to diagnose in real time whether a person is being effectively trained or the brain function is approaching normal.

Provision of the intra-brain information 71 also provides the following advantages. (1) It is possible to support judgment as to whether a person is concentrating on listening to a lesson, etc. (2) It is possible to which part of the brain causes the failure to track a speech in a non-native language (e.g., English for Japanese people). (3) It is possible to help examine which part of the brain causes a sound to be heard wrong. (4) It is possible to examine a cause of auditory hallucination, ear ringing, etc. from the perspective of brain activity and support neurofeedback treatment.

A supplementary description will be given of the estimation precision of the sound estimation model of the embodiment. The comparison image shown in Fig. 6 shows an experiment result of the estimation system 10 and compares the waveform of the original sound presented to a healthy individual and the waveform of the recognized sound estimated from the brain wave of the healthy individual. As already described, the comparison image shows the waveform of the original sound in a broken line and shows the waveform of the recognized sound in a solid line, for each of "Ah", "Ee", and noise.

We have evaluated a gap between the waveform of the original sound and the waveform of the recognized sound by calculating a coefficient of determination R2. When the waveforms match completely, R2=1. Our experiment revealed that R2 in the case of the sound "Ah" is "0.983", R2 in the case of the sound "Ee" is "0.957", and R2 in the case of noise is "0.997". Even at R2 of about 0.7, the waveforms are similar. It can therefore be said that the precision of estimation by the sound estimation model, i.e., the precision of sound resynthesis using the brain wave is quite high.

Even if R2 is high, however, it would be meaningless if the sound actually synthesized based on the recognized sound information (i.e., the sound played back by the playback unit 58) is not heard as being the same as the original sound. We have therefore checked, through experiments, to find a measure of the coefficient of determination R2 that makes the sound synthesized based on the recognized sound information heard as being the same as the original sound.

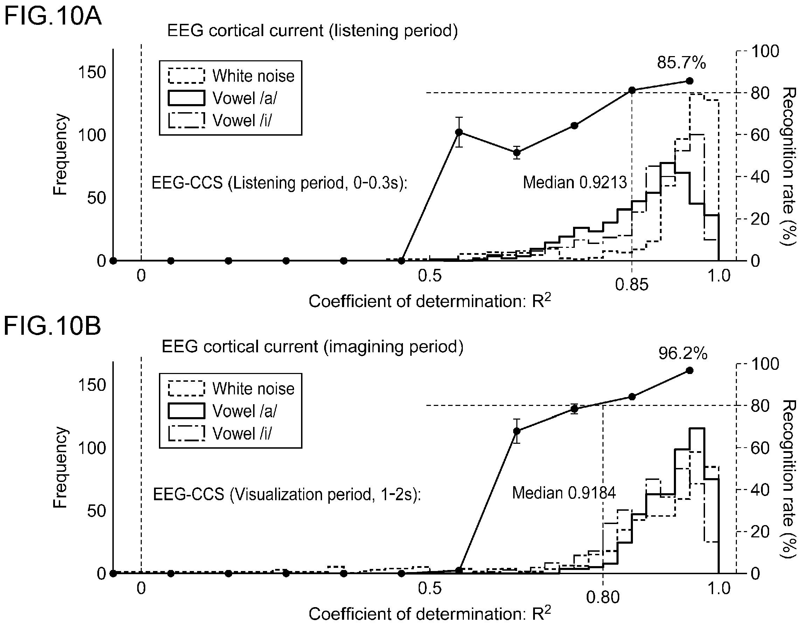

Figs. 10A and 10B are graphs showing results of experiments. Fig. 10A shows a result yielded when the recognized sound is synthesized (played back) from the brain wave of the subject who is hearing the original sound with the ears. Fig. 10B shows a result yielded when the recognized sound is synthesized (played back) from the brain wave of the subject who heard the original sound with the ears and is remembering the sound. The horizontal axis represents the coefficient of determination R2 indicating a gap between the waveform of the original sound and the waveform of the recognized sound. The polygonal line graph shows a proportion in which the sound synthesized based on the recognized sound information is recognized as being the same as the original sound.

The experimental result revealed that R2 of about 0.8-0.85 is necessary for the recognition rate indicated by the polygonal line graph to be 80% or higher. Further, the histogram of Fig. 10A and Fig. 10B show the R2 distribution of actual data. Fig. 10A and Fig. 10B show that data showing R2 of 0.8 or higher occupies 77.2%-79.35 or higher of the entirety, demonstrating that the precision of estimation by the sound estimation model is high as a whole.

Described above is an explanation based on an exemplary embodiment. The embodiment is illustrative, and it will be understood by those skilled in the art that various modifications to constituting elements and processes could be developed and that such modifications are also within the scope of the present disclosure. Variations will be described below.

A first variation will be described. In the embodiment described above, the sound estimation model is realized by a neural network. In a variation, a mathematical mode or a function as the sound estimation model may be generated by other machine learning schemes. For example, the learning unit 32 of the model generation apparatus 16 may generates a sound estimation model that estimates the recognized sound (e.g., the category of the recognized sound) of the subject based on the input signal source data, according to a scheme such as sparse logistic regression (SLR) or support vector machine (SVM).

A description will be given of a second variation. In the embodiment described above, VBMEG is used to estimate a signal source, but a signal source may be estimated by other schemes. For example, a signal source may be estimated by using standardized low-resolution brain electromagnetic tomography (sLORETA). sLORETA is a scheme for brain function imaging analysis and is an analysis scheme for depicting the intra-brain activity according to bran wave or magnetoencephalography by superimposing it on a brain atlas (i.e., standard brain).

A description will be given of a third embodiment. In the embodiment described above, the fMRI apparatus 14 is used to identify a signal source of the use's brain wave (i.e., the brain activity), but the embodiment may be configured not to include the fMRI apparatus 14. For example, the correspondence between the mode of brain wave and the signal source may be hypothesized or identified based on the anatomical knowledge and/or the shape of the skull bone estimated from the three-dimensional position of the electrode of the electroencephalograph 12. In this case, the fMRI apparatus 14 will not be necessary. The signal source estimation unit 28 may estimate a signal source based on the above correspondence.

A description will be given of a fourth variation. In the embodiment described above, the first subject and the second subject are assumed to be the same person. In a variation, the first subject and the second subject may be different persons. For example, the first subject may be a healthy person (a person who can understand the sound and express his or her intention), and the second subject may be a person who finds it difficult to express his or her intention (i.e., communicate) such as a person having hearing difficulties, a person in a vegetative state, a locked-in patient, etc. In this case, the sound estimated to be recognized by the person, as the second subject, who finds it difficult to express his or her intention, may be synthesized and reproduced by using the sound estimation model created based on the brain wave (and the signal source thereof) of the healthy person as the first subject.

A description will be given of a fifth variation. The technology described above in the embodiment may be applied to realize an information processing apparatus (the estimation apparatus 18) adapted to the second subject recalling (i.e., bringing to mind or remembering) an arbitrary sound and configured to estimate the sound recalled by the second subject. The model storage unit 40 of the estimation apparatus 18 of this variation may store the data for the sound estimation model build by machine learning that uses, as training data, information on a plurality of types of sound (e.g., "Ah" through "Nn" in Japanese) and information relating to the signal source of the brain wave of the first subject presented with each of the plurality of types of sound. The brain wave acquisition unit 48 of the estimation apparatus 18 may acquire the brain wave of the second subject who recalls an arbitrary sound (e.g., one of "Ah" through "Nn"). The recognized sound estimation unit 52 of the estimation apparatus 18 may input the information relating to signal source of the brain wave of the second subject to the sound estimation model and acquire the information on the sound (recalled sound) output from the sound estimation model as being estimated to be recalled by the second subject. According to this mode, the information on the sound itself estimated to be remembered by the subject (the second subject) can be generated.

The estimation apparatus 18 of the fifth variation may process and output the recalled sound as it similarly processes and outputs the recognized sound of the embodiment. For example, (1) the playback unit 58 of the estimation apparatus 18 may play back the sound indicated by the information on the recalled sound. Alternatively, (2) the sound estimation model may record information relating to the impact of each of the plurality of regions in the brain of the second subject on the recalled sound. The intra-brain information generation unit 62 may refer to the information recorded in the sound estimation model and generate intra-brain information indicating the impact of each of the regions in the brain of the second subject on the recalled sound.

A recalled sound is a sound (including a speech) that comes to the mind of the second subject and includes a sound that the second subject does not present outside explicitly. Further, the recalled sound is inclusive of a sound (including a speech) that comes to the mind of the second subject unconsciously. In other words, it is possible, according to the estimation system 10 of the fifth variation, to obtain information on the sound that comes to the mind of the second subject even if the second subject does not think about it consciously. When the second subject is mainly thinking in a public principle manifested outside and entertaining inner thoughts in the back of his or her head, for example, information that includes both the sound relating to the public principle and the sound relating to the inner thoughts can be obtained.

A description will be given of a sixth variation. In the embodiment and the variations, the brain wave is used as a signal indicating the brain activity of the first subject and the second subject. In this variation, magnetoencephalography may be used as a signal indicating the brain activity of the first subject and the second subject. In this case, the estimation system 10 may be provided with, in place of the electroencephalograph 12 shown in Fig. 2, a magnetoencephalometer that measures the magnetic field produced by the electric activity of the brain. The brain activity acquisition unit of the model generation apparatus 16 and the estimation apparatus 18 may acquire data for magnetoencephalography measured by the magnetoencephalometer. The signal source estimation unit of the model generation apparatus 16 and the estimation apparatus 18 may estimate a signal source of the magnetoencephalography based on the mode of magnetoencephalography.

In an alternative mode of the sixth variation, the result of measurement by a NIRS brain measuring apparatus (optical topography (registered trademark)) may be used as a signal indicating the brain activity of the first subject and the second subject. The NIRS brain measuring apparatus may measure a signal that serves as an indicator of the blood flow rate, increase or decrease in hemoglobin, oxygen change amount, etc. in the brain cortex. In this case, the estimation system 10 may be provided with a NIRS brain measuring apparatus in place of the electroencephalograph 12 shown in Fig. 2. The brain activity acquisition unit of the model generation apparatus 16 and the estimation apparatus 18 may acquire data for a signal measured by the NIRS brain measuring apparatus. The signal source estimation unit of the model generation apparatus 16 and the estimation apparatus 18 may, based on the mode of the signal measured by the NIRS brain measuring apparatus, estimate a signal source of that signal.

Any combination of the embodiment and the variation described above will also be useful as an embodiment of the present disclosure. A new embodiment created by a combination will provide the respective advantages of the embodiment and the variation as combined. It will be understood by a skilled person that the functions that the constituting elements recited in the claims should achieve are implemented either alone or in combination by the constituting elements shown in the embodiments and the variations.

### [INDUSTRIAL APPLICABILITY]

The technology of the present disclosure can be applied to apparatuses and systems for estimating a sound recognized or remembered by a person.

### [REFERENCE SIGNS LIST]

10 estimation system, 18 estimation apparatus, 26 brain wave acquisition unit, 28 signal source estimation unit, 40 model storage unit, 48 brain wave acquisition unit, 50 signal source estimation unit, 52 recognized sound estimation unit, 54 recognized sound storage unit, 58 playback unit, 60 image generation unit, 62 intra-brain information generation unit

## Claims

1. An information processing apparatus capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a signal indicating a brain activity of a subject, information on a sound estimated to be recognized by the subject, the information processing apparatus comprising:
a brain activity acquisition unit that acquires a signal indicating a brain activity of a second subject presented with the predetermined sound;
a signal source estimation unit that estimates, based on a mode of the signal indicating the brain activity acquired by the brain activity acquisition unit, a signal source of the signal indicating the brain activity, from among a plurality of regions in a brain of the second subject; and
a recognized sound acquisition unit that inputs the information relating to the signal source estimated by the signal source estimation unit to the model and acquires information, output from the model, on a recognized sound estimated to be recognized by the second subject.

2. The information processing apparatus according to claim 1, further comprising:
a playback unit that plays back the sound indicated by the information on the recognized sound acquired by the recognized sound acquisition unit.

3. The information processing apparatus according to claim 1 or 2, further comprising:
an intra-brain information generation unit, wherein
the model, to which the information relating to the signal source is input, records information on an impact of each of the plurality of regions in the brain of the second subject on the recognized sound, and
the intra-brain information generation unit refers to the information recorded in the model and generates intra-brain information indicating the impact of each of the plurality of regions in the brain of the second subject on the recognized sound.

4. The information processing apparatus according to any one of claims 1 through 3, further comprising:
an image generation unit that generates an image indicating a waveform of the predetermined sound and a waveform of the recognized sound.

5. An information processing apparatus capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a signal indicating a brain activity of a subject, information on a sound estimated to be recognized by the subject, the information processing apparatus comprising:
a brain activity acquisition unit that acquires a signal indicating a brain activity of a second subject recalling an arbitrary sound;
a signal source estimation unit that estimates, based on a mode of the signal indicating the brain activity acquired by the brain activity acquisition unit, a signal source of the signal indicating the brain activity, from among a plurality of regions in a brain of the second subject; and
a sound acquisition unit that inputs the information relating to the signal source estimated by the signal source estimation unit to the model and acquires information, output from the model, on a sound estimated to be recalled by the second subject.

6. An information processing method implemented on a computer capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a signal indicating a brain activity of a subject, information on a sound estimated to be recognized by the subject, the method comprising:
acquiring a signal indicating a brain activity of a second subject presented with the predetermined sound;
estimating, based on a mode of the signal indicating the brain activity acquired, a signal source of the signal indicating the brain activity, from among a plurality of regions in a brain of the second subject; and
inputting the information relating to the signal source estimated to the model and acquiring information, output from the model, on a recognized sound estimated to be recognized by the second subject.

7. An information processing method implemented on a computer capable of accessing a model storage unit that stores a model built by machine learning, using, as training data, information on a predetermined sound and information relating to a signal source of a signal indicating a brain activity of a first subject presented with the predetermined sound, the model outputting, based on input information relating to a signal source of a signal indicating a brain activity of a subject, information on a sound estimated to be recognized by the subject, the method comprising:
acquiring a signal indicating a brain activity of a second subject recalling an arbitrary sound;
estimating, based on a mode of the signal indicating the brain activity acquired, a signal source of the signal indicating the brain activity, from among a plurality of regions in a brain of the second subject; and
inputting the information relating to the signal source estimated to the model and acquiring information, output from the model, on a sound estimated to be recalled by the second subject.
